# FASCICULE DE BREVET EUROPEEN

(11) **EP 0 468 875 B1**
(45) Date de publication et mention de la délivrance du brevet: **28.06.1995**
(21) Numéro de dépôt: 91402036.7
(22) Date de dépôt: 23.07.1991
(51) Int. Cl.: A61L 15/44

(54) **Système matriciel autoadhésif pour la libération prolongée du piribédil par voie transcutanée**
Selbstklebendes Matrixsystem für transdermal gesteuerte Abgabe von Piribedil
Autoadhesive matrix system for transdermally controlled release of piribedil

(30) Priorité: 23.07.1990 FR 9009361
(43) Date de publication de la demande: 29.01.1992
(73) Titulaire: ADIR ET COMPAGNIE, 92415 Courbevoie Cédex (FR)
(72) Inventeur: Cuine, Alain, F-45800 Saint Jean de Braye (FR); Rollot, Jean-Marc, F-45000 Orleans (FR); Rault, Isabelle, F-45170 Saint Lye la Forêt (FR); Arnaud, Françoise, F-92250 La Garenne Colombes (FR); Evrard, Yvonne, F-92500 Rueil Malmaison (FR); Chezaubernard, Claire, F-75003 Paris (FR)

(56) Documents cités:
- EP-A- 0 084 993
- EP-A- 0 196 769
- EP-A- 0 304 381

## Description

La présente invention a pour objet un système matriciel autoadhésif pour la libération prolongée du piribédil qui assure la diffusion continue et progressive du principe actif par voie transcutanée.

Le piribédil, composé de formule **(I)** : 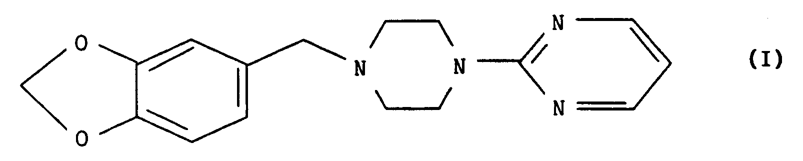
est un agoniste dopaminergique qui stimule les récepteurs à la dopamine et les voies dopaminergiques cérébrales utilisé dans le traitement des troubles psychocomportementaux de la sénescence cérébrale et des séquelles récentes ou tardives des accidents vasculaires cérébraux, du tremblement de repos extrapyramidal et dans les manifestations de l'artérite des membres inférieurs.

Le piribédil était jusqu'alors administré par voie orale ou injectable.

L'administration de ce principe actif par voie transcutanée présente l'avantage au plan pharmacocinétique d'une obtention rapide de taux plasmatiques élevés et constants, au plan métabolique d'éviter la métabolisation importante du principe actif par effet de premier passage hépatique et enfin au plan clinique de diminuer les doses administrées en améliorant l'efficacité, l'acceptabilité clinique et l'observance par une seule application non traumatisante.

Les possibilités d'administration d'un médicament à travers la peau dépendent de différents facteurs. En particulier le composé ne doit pas altérer la peau d'une manière quelconque à la suite d'un contact prolongé et provoquer irritations, allergies ou sensibilisations, et doit pouvoir traverser une surface de peau assez petite à un débit de diffusion suffisant pour l'obtention de taux plasmatiques adaptés aux besoins thérapeutiques.

Or, le piribédil satisfait à toutes ces exigences.

Plus spécifiquement, la présente invention a pour objet un système matriciel autoadhésif qui permet la libération prolongée du piribédil et son passage par voie transcutanée à travers une surface de peau déterminée, pendant une période prolongée et à un débit suffisant pour provoquer la stimulation des récepteurs dopaminergiques.

Ainsi le système transdermique de piribédil permet d'améliorer les troubles psychocomportementaux de la sénescence cérébrale en particulier les fonctions mnésiques, la capacité de mémorisation, la vigilance et la capacité d'adaptation à l'environnement. Il est également utile pour le traitement des accidents vasculaires cérébraux, du tremblement de repos extrapyramidal, des accidents ischémiques rétiniens, des troubles cochléovestibulaires d'origine ischémique et enfin dans les manifestations de l'artérite des membres inférieurs.

Depuis quelques années de nombreux brevets décrivent des systèmes pour l'administration de médicaments par voie transdermique à la circulation générale.

En particulier, il existe des systèmes autoadhésifs dans lesquels le principe actif est directement dissous dans l'adhésif.

D'autre part , il existe des systèmes matriciels dans lesquels le principe actif est à la fois dissous et dispersé à l'état particulaire ; la préparation est alors rendue adhésive par l'enduction d'un adhésif en surface ou par un anneau situé autour de la matrice.

Le système transdermique de libération prolongée du piribédil présente l'avantage d'allier les deux caractéristiques précedentes d'autoadhésion et de matrice contenant le piribédil dispersé dans une même base silicone élastomère adhésive.

Les silicones élastomères présentent l'avantage, par rapport aux autres polymères de ne pas contenir d'antioxydants, plastifiants et stabilisants.

En outre, ce système silicone élastomère adhésif est nouveau.

Enfin, la libération du piribédil se fait de façon continue et constante pendant toute la durée d'application du système à la peau.

Le dispositif transdermique est constitué d'un film support occlusif en complexe polyéthylène/aluminium/polyester enduit d'une matrice silicone élastomère autoadhésive recouverte d'un film protecteur pelable.

La matrice constitue un réseau polymérique susceptible de dissoudre le piribédil ayant un comportement autoadhésif permettant de maintenir en place le système pendant une période comprise entre 1 et 7 jours.

Le squelette polymère est constitué d'un élastomère silicone de qualité médicale de formule générale : 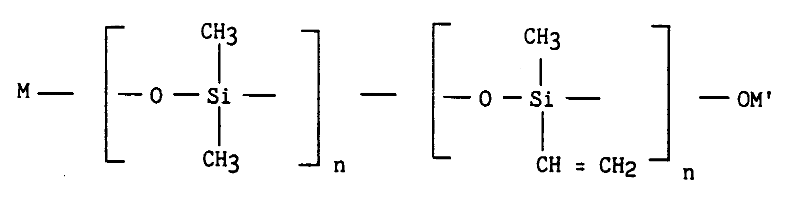
dans laquelle
M et M′ sont les groupements :

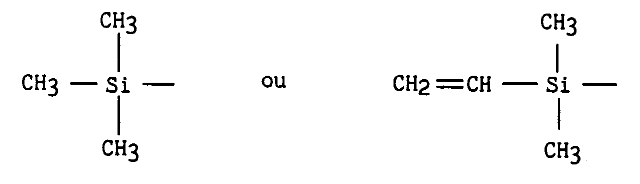

La réticulation de ce polysiloxane est effectuée à chaud par hydrosilylation à l'aide de polysiloxane à fonction SiH en présence de platine comme catalyseur, en étuve chauffée ou en utilisant un rayonnement infrarouge.

Le pouvoir autoadhésif de la matrice est conféré par l'incorporation d'un deuxième silicone élastomère en solution dans un solvant organique approprié comme par exemple l'heptane, l'hexane ou le trichlorotrifluoroéthane à une concentration déterminée.

Cet adhésif est constitué d'un mélange en proportions définies d'un polymère à basse viscosité (12000 à 15000 mPa.s) possèdant des groupements silanols terminaux Si-OH et d'un silicone résineux triméthylsiloxysilicate tridimensionnel.

En solution dans un solvant approprié, l'adhésif est produit par condensation entre les groupements silanols des deux polymères. Les propriétés adhésives sont révélées par évaporation du solvant.

Les propriétés autoadhésives de la matrice peuvent être augmentées par l'utilisation conjointe d'une matrice élastomère présentant une certaine adhésivité par elle-même et d'un silicone élastomère adhésif dans un solvant organique approprié.

Des promoteurs d'absorption peuvent enfin être ajoutés à la matrice. Ces promoteurs d'absorption peuvent être du lauryl sulfate de sodium, du propylèneglycol ou du diméthylisosorbide.

Les quantités du piribédil dans la matrice varient en fonction des doses nécessaires pour un type de patient particulier et sont comprises entre 10 et 500 mg.

Les polysiloxanes utilisés sont biologiquement et pharmaceutiquement acceptables, non allergènes, insolubles dans l'organisme et non irritants pour les tissus avec lesquels ils sont en contact.

Les exemples suivants illustrent l'invention mais ne la limitent en aucune façon.

### EXEMPLE 1 :

12 g de piribédil sont incorporés progressivement à 42 g du silicone élastomère de qualité médicale (MDX4-4210) contenant son agent réticulant sous agitation lente. L'ensemble obtenu est ensuite homogénéisé par passage sur un broyeur à trois cylindres.

66 g du silicone élastomère adhésif de qualité médicale (en solution à 60 % dans l'hexane) sont alors introduits au mélange précédent.

Lorsque la viscosité du mélange a atteint la valeur adéquate par évaporation de l'hexane, le film support occlusif en complexe polyéthylène/aluminium/polyester est enduit de ce mélange au moyen d'un étaleur à épaisseur réglable.

La réticulation est alors réalisée en étuve ventilée pendant 1 h à 80°C.

Un film protecteur pelable en polychlorure de vinyle est appliqué sur le système adhésif réticulé.

La matrice autoadhésive est alors découpée de façon à obtenir des dispositifs transdermiques ronds de surface égale à 20 cm² ce qui correspond à 40 mg de principe actif par système.

### EXEMPLE 2 :

12 g de piribédil sont incorporés à 42 g du silicone élastomère de qualité médicale (MDX4-4210) contenant son agent réticulant sous agitation lente en présence de 0,6 g de lauryl sulfate de sodium. La matrice est obtenue de la même façon que celle décrite dans l'exemple 1.

La matrice autoadhésive est alors découpée de façon à obtenir des dispositifs transdermiques carrés de surface égale à 20 cm² ce qui correspond à 40 mg de principe actif par système.

### EXEMPLE 3 :

La réalisation du système transdermique est identique à celle décrite dans l'exemple 1.

Un film protecteur pelable en polyester siliconé est alors appliqué sur le système adhésif réticulé.

La matrice autoadhésive est enfin découpée de façon à obtenir des dispositifs transdermiques ronds de surface égale à 80 cm² ce qui correspond à 160 mg de principe actif par système.

### EXEMPLE 4 :

4 g de piribédil micronisé sont incorporés à 10 g d'heptane. Le mélange est soumis aux ultrasons. Lorsque le piribédil est parfaitement dispersé, 20 g de silicone élastomère de qualité médicale contenant son agent réticulant (MDX4-4210) sont ajoutés au mélange et l'ensemble est homogénéisé.

14 g de silicone élastomère adhésif de qualité médicale (en solution à 74 % dans l'heptane) et 2 g de propylèneglycol sont alors introduits au mélange précédent. L'enduction est identique à celle décrite dans l'exemple 1.

La réticulation est réalisée en quelques minutes, après passage sous un dispositif à rayonnement infrarouge.

Après refroidissement de la matrice, un film protecteur pelable en polyester est appliqué sur le système adhésif réticulé.

La matrice est alors découpée de façon à obtenir des dispositifs transdermiques carrés à coins arrondis de 20 cm², correspondant à 80 mg de piribédil par système.

### ETUDE PHARMACOLOGIQUE DES DERIVES DE L'INVENTION

### EXEMPLE 5 :

Pour déterminer l'accessibilité du piribédil par voie transdermique, des études in vivo chez l'homme sain ont été effectuées.

L'absorption du piribédil par voie transcutanée a été déterminée par dosage GC-MS des concentrations plasmatiques du principe actif lors de l'application du dispositif décrit dans l'exemple 3 pendant 72 heures.

Les résultats de l'absorption de piribédil sont indiqués dans le schéma I.

L'allure de la courbe du schéma I montre que pour un dispositif transdermique donné, la concentration plasmatique de piribédil est constante pendant les 72 heures d'application du dispositif et s'établit très rapidement.

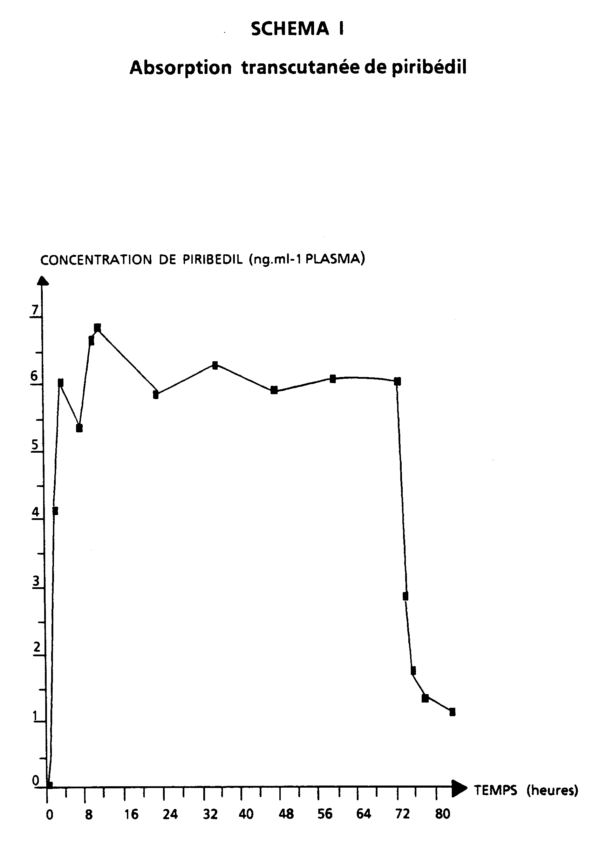

## Revendications (Revendications pour l'(les) Etat(s) contractant(s) suivant(s): AT, BE, CH, DE, DK, FR, GB, IT, LI, LU, NL, SE)

1. Système matriciel autoadhésif pour la libération prolongée du piribédil par voie transcutanée caractérisée en ce qu'il comprend un film support occlusif enduit d'une matrice autoadhésive recouverte d'un film protecteur pelable.

2. Système matriciel autoadhésif de piribédil selon la revendication 1 caractérisé en ce que le squelette polymère de la matrice est constitué d'un silicone élastomère de qualité médicale dans lequel est dispersé le piribédil et éventuellement des promoteurs d'absorption.

3. Système matriciel autoadhésif de piribédil selon la revendication 1 caractérisé en ce que la matrice est rendue autoadhésive par l'incorporation d'un deuxième silicone élastomère en solution dans un solvant organique.

4. Système matriciel autoadhésif de piribédil selon la revendication 1 caractérisé en ce que la réticulation du squelette polymère de la matrice est réalisée par passage sous un dispositif à rayonnement infrarouge.

5. Système matriciel autoadhésif de piribédil selon la revendication 1 permettant la libération prolongée du piribédil pendant une période comprise entre 1 et 7 jours.

6. Système matriciel autoadhésif de piribédil selon l'une quelconque des revendications 1 à 5 utile dans le traitement des troubles psychocomportementaux de la sénescence cérébrale, des accidents vasculaires cérébraux, du tremblement de repos extrapyramidal, des accidents ischémiques rétiniens, des troubles cochléovestibulaires d'origine ischémique et des manisfestations de l'artérite des membres inférieurs.

## Revendications (Revendications pour l'(les) Etat(s) contractant(s) suivant(s): ES)

1. Système matriciel autoadhésif pour la libération prolongée du piribédil par voie transcutanée caractérisée en ce qu'il comprend un film support occlusif enduit d'une matrice autoadhésive recouverte d'un film protecteur pelable.

2. Système matriciel autoadhésif de piribédil selon la revendication 1 caractérisé en ce que le squelette polymère de la matrice est constitué d'un silicone élastomère de qualité médicale dans lequel est dispersé le piribédil et éventuellement des promoteurs d'absorption.

3. Système matriciel autoadhésif de piribédil selon la revendication 1 caractérisé en ce que la matrice est rendue autoadhésive par l'incorporation d'un deuxième silicone élastomère en solution dans un solvant organique.

4. Système matriciel autoadhésif de piribédil selon la revendication 1 caractérisé en ce que la réticulation du squelette polymère de la matrice est réalisée par passage sous un dispositif à rayonnement infrarouge.

5. Système matriciel autoadhésif de piribédil selon la revendication 1 permettant la libération prolongée du piribédil pendant une période comprise entre 1 et 7 jours.

6. Système matriciel autoadhésif de piribédil selon l'une quelconque des revendications 1 à 5 utile dans le traitement des troubles psychocomportementaux de la sénescence cérébrale, des accidents vasculaires cérébraux, du tremblement de repos extrapyramidal, des accidents ischémiques rétiniens, des troubles cochléovestibulaires d'origine ischémique et des manisfestations de l'artérite des membres inférieurs.

## Revendications (Revendications pour l'(les) Etat(s) contractant(s) suivant(s): GR)

1. Système matriciel autoadhésif pour la libération prolongée du piribédil par voie transcutanée caractérisée en ce qu'il comprend un film support occlusif enduit d'une matrice autoadhésive recouverte d'un film protecteur pelable.

2. Système matriciel autoadhésif de piribédil selon la revendication 1 caractérisé en ce que le squelette polymère de la matrice est constitué d'un silicone élastomère de qualité médicale dans lequel est dispersé le piribédil et éventuellement des promoteurs d'absorption.

3. Système matriciel autoadhésif de piribédil selon la revendication 1 caractérisé en ce que la matrice est rendue autoadhésive par l'incorporation d'un deuxième silicone élastomère en solution dans un solvant organique.

4. Système matriciel autoadhésif de piribédil selon la revendication 1 caractérisé en ce que la réticulation du squelette polymère de la matrice est réalisée par passage sous un dispositif à rayonnement infrarouge.

5. Système matriciel autoadhésif de piribédil selon la revendication 1 permettant la libération prolongée du piribédil pendant une période comprise entre 1 et 7 jours.

6. Système matriciel autoadhésif de piribédil selon l'une quelconque des revendications 1 à 5 utile dans le traitement des troubles psychocomportementaux de la sénescence cérébrale, des accidents vasculaires cérébraux, du tremblement de repos extrapyramidal, des accidents ischémiques rétiniens, des troubles cochléovestibulaires d'origine ischémique et des manisfestations de l'artérite des membres inférieurs.

## Claims (Claims for the following Contracting State(s): AT, BE, CH, DE, DK, FR, GB, IT, LI, LU, NL, SE)

1. Self-adhesive matrix system for the sustained release of piribedil by the transcutaneous route, characterised in that it comprises an occlusive support film which is coated with a self-adhesive matrix covered with a removable protective film.

2. Self-adhesive piribedil matrix system according to claim 1, characterised in that the polymer skeleton of the matrix consists of a medical grade silicone elastomer in which is dispersed the piribedil and, optionally, absorption promoters.

3. Self-adhesive piribedil matrix system according to claim 1, characterised in that the matrix is rendered self-adhesive by the incorporation of a second silicone elastomer in solution in an organic solvent.

4. Self-adhesive piribedil matrix system according to claim 1, characterised in that the polymer skeleton of the matrix is cross-linked by passage through an infra-red radiation device.

5. Self-adhesive piribedil matrix system according to claim 1 allowing the sustained release of piribedil over a period of from 1 to 7 days.

6. Self-adhesive piribedil matrix system according to any one of claims 1 to 5 which can be used in the treatment of psychobehavioural disorders of cerebral senescence, cerebrovascular accidents, resting extrapyramidal tremor, ischaemic retinal accidents, cochleovestibular disorders of ischaemic origin and symptoms of arteritis of the lower limbs.

## Claims (Claims for the following Contracting State(s): ES)

1. Self-adhesive matrix system for the sustained release of piribedil by the transcutaneous route, characterised in that it comprises an occlusive support film which is coated with a self-adhesive matrix covered with a removable protective film.

2. Self-adhesive piribedil matrix system according to claim 1, characterised in that the polymer skeleton of the matrix consists of a medical grade silicone elastomer in which is dispersed the piribedil and, optionally, absorption promoters.

3. Self-adhesive piribedil matrix system according to claim 1, characterised in that the matrix is rendered self-adhesive by the incorporation of a second silicone elastomer in solution in an organic solvent.

4. Self-adhesive piribedil matrix system according to claim 1, characterised in that the polymer skeleton of the matrix is cross-linked by passage through an infra-red radiation device.

5. Self-adhesive piribedil matrix system according to claim 1 allowing the sustained release of piribedil over a period of from 1 to 7 days.

6. Self-adhesive piribedil matrix system according to any one of claims 1 to 5 which can be used in the treatment of psychobehavioural disorders of cerebral senescence, cerebrovascular accidents, resting extrapyramidal tremor, ischaemic retinal accidents, cochleovestibular disorders of ischaemic origin and symptoms of arteritis of the lower limbs.

## Claims (Claims for the following Contracting State(s): GR)

1. Self-adhesive matrix system for the sustained release of piribedil by the transcutaneous route, characterised in that it comprises an occlusive support film which is coated with a self-adhesive matrix covered with a removable protective film.

2. Self-adhesive piribedil matrix system according to claim 1, characterised in that the polymer skeleton of the matrix consists of a medical grade silicone elastomer in which is dispersed the piribedil and, optionally, absorption promoters.

3. Self-adhesive piribedil matrix system according to claim 1, characterised in that the matrix is rendered self-adhesive by the incorporation of a second silicone elastomer in solution in an organic solvent.

4. Self-adhesive piribedil matrix system according to claim 1, characterised in that the polymer skeleton of the matrix is cross-linked by passage through an infra-red radiation device.

5. Self-adhesive piribedil matrix system according to claim 1 allowing the sustained release of piribedil over a period of from 1 to 7 days.

6. Self-adhesive piribedil matrix system according to any one of claims 1 to 5 which can be used in the treatment of psychobehavioural disorders of cerebral senescence, cerebrovascular accidents, resting extrapyramidal tremor, ischaemic retinal accidents, cochleovestibular disorders of ischaemic origin and symptoms of arteritis of the lower limbs.

## Patentansprüche (Patentansprüche für folgende(n) Vertragsstaat(en): AT, BE, CH, DE, DK, FR, GB, IT, LI, LU, NL, SE)

1. Selbstklebendes Matrixsystem für die verzögerte Abgabe von Piribedil auf transkutanem Wege, **dadurch gekennzeichnet**, daß es einen abschließenden Filmträger umfaßt, der mit einer selbstklebenden Matrix beschichtet ist, die mit einem abziehbaren Schutzfilm bedeckt ist.

2. Selbstklebendes Piribedil-Matrixsystem nach Anspruch 1, **dadurch gekennzeichnet**, daß das Polymergerüst der Matrix aus einem Siliconelastomeren von medizinischer Qualität gebildet ist, in dem Pribedil und gegebenenfalls die Absorption fördernde Mittel dispergiert sind.

3. Selbstklebendes Piribedil-Matrixsystem nach Anspruch 1, **dadurch gekennzeichnet**, daß die Matrix durch Einarbeiten eines zweiten, in einem organischen Lösungsmittel gelösten Siliconelastomeren selbstklebend gemacht ist.

4. Selbstklebendes Piribedil-Matrixsystem nach Anspruch 1, **dadurch gekennzeichnet**, daß die Vernetzung des Polymergesrüsts der Matrix durch Hindurchführen unter einer Infrarotbestrahlungseinrichtung bewirkt worden ist.

5. Selbstklebendes Piribedil-Matrixsystem nach Anspruch 1 für die verzögerte Abgabe von Piribedil während einer Zeitdauer zwischen 1 und 7 Tagen.

6. Selbstklebendes Piribedil-Matrixsystem nach einem der Ansprüche 1 bis 5 für die Behandlung von psychischen Verhaltensstörungen beim Altern des Gehirns, von Gehirngefäßvorfällen, des extrapyramidalen Ruhezitterns, von ischämischen Retinavorfällen, von Ohrschneckenvorhof-bezüglichen Störungen ischämischen Ursprungs und Arterienerkrankungen der unteren Gliedmaßen.

## Patentansprüche (Patentansprüche für folgende(n) Vertragsstaat(en): ES)

1. Selbstklebendes Matrixsystem für die verzögerte Abgabe von Piribedil auf transkutanem Wege, **dadurch gekennzeichnet**, daß es einen abschließenden Filmträger umfaßt, der mit einer selbstklebenden Matrix beschichtet ist, die mit einem abziehbaren Schutzfilm bedeckt ist.

2. Selbstklebendes Piribedil-Matrixsystem nach Anspruch 1, **dadurch gekennzeichnet**, daß das Polymergerüst der Matrix aus einem Siliconelastomeren von medizinischer Qualität gebildet ist, in dem Pribedil und gegebenenfalls die Absorption fördernde Mittel dispergiert sind.

3. Selbstklebendes Piribedil-Matrixsystem nach Anspruch 1, **dadurch gekennzeichnet**, daß die Matrix durch Einarbeiten eines zweiten, in einem organischen Lösungsmittel gelösten Siliconelastomeren selbstklebend gemacht ist.

4. Selbstklebendes Piribedil-Matrixsystem nach Anspruch 1, **dadurch gekennzeichnet**, daß die Vernetzung des Polymergesrüsts der Matrix durch Hindurchführen unter einer Infrarotbestrahlungseinrichtung bewirkt worden ist.

5. Selbstklebendes Piribedil-Matrixsystem nach Anspruch 1 für die verzögerte Abgabe von Piribedil während einer Zeitdauer zwischen 1 und 7 Tagen.

6. Selbstklebendes Piribedil-Matrixsystem nach einem der Ansprüche 1 bis 5 für die Behandlung von psychischen Verhaltensstörungen beim Altern des Gehirns, von Gehirngefäßvorfällen, des extrapyramidalen Ruhezitterns, von ischämischen Retinavorfällen, von Ohrschneckenvorhof-bezüglichen Störungen ischämischen Ursprungs und Arterienerkrankungen der unteren Gliedmaßen.

## Patentansprüche (Patentansprüche für folgende(n) Vertragsstaat(en): GR)

1. Selbstklebendes Matrixsystem für die verzögerte Abgabe von Piribedil auf transkutanem Wege, **dadurch gekennzeichnet**, daß es einen abschließenden Filmträger umfaßt, der mit einer selbstklebenden Matrix beschichtet ist, die mit einem abziehbaren Schutzfilm bedeckt ist.

2. Selbstklebendes Piribedil-Matrixsystem nach Anspruch 1, **dadurch gekennzeichnet**, daß das Polymergerüst der Matrix aus einem Siliconelastomeren von medizinischer Qualität gebildet ist, in dem Pribedil und gegebenenfalls die Absorption fördernde Mittel dispergiert sind.

3. Selbstklebendes Piribedil-Matrixsystem nach Anspruch 1, **dadurch gekennzeichnet**, daß die Matrix durch Einarbeiten eines zweiten, in einem organischen Lösungsmittel gelösten Siliconelastomeren selbstklebend gemacht ist.

4. Selbstklebendes Piribedil-Matrixsystem nach Anspruch 1, **dadurch gekennzeichnet**, daß die Vernetzung des Polymergesrüsts der Matrix durch Hindurchführen unter einer Infrarotbestrahlungseinrichtung bewirkt worden ist.

5. Selbstklebendes Piribedil-Matrixsystem nach Anspruch 1 für die verzögerte Abgabe von Piribedil während einer Zeitdauer zwischen 1 und 7 Tagen.

6. Selbstklebendes Piribedil-Matrixsystem nach einem der Ansprüche 1 bis 5 für die Behandlung von psychischen Verhaltensstörungen beim Altern des Gehirns, von Gehirngefäßvorfällen, des extrapyramidalen Ruhezitterns, von ischämischen Retinavorfällen, von Ohrschneckenvorhof-bezüglichen Störungen ischämischen Ursprungs und Arterienerkrankungen der unteren Gliedmaßen.
